(19) Europäisches Patentamt / European Patent Office / Office européen des brevets

(11) **EP 3 059 590 A1**

(12) **EUROPEAN PATENT APPLICATION**

(43) Date of publication:
24.08.2016 Bulletin 2016/34

(51) Int Cl.:
*G01N 33/543* (2006.01)          *G01N 33/569* (2006.01)
*G01N 33/68* (2006.01)

(21) Application number: 15155483.9

(22) Date of filing: 17.02.2015

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
• **Norwegian Institute of Public Health**
  **0403 Oslo (NO)**
• **Armauer Hansen Research Insitute**
  **Addis Ababa (ET)**

(72) Inventors:
• **HOLM-HANSEN, Carol Joanne Church**
  **0403 Olso (NO)**
• **ASEFFA, Abraham**
  **Addis Ababa (ET)**
• **ABEBE, Markos**
  **Addis Ababa (ET)**

(74) Representative: **Arends, William Gerrit**
  **Marks & Clerk LLP**
  **90 Long Acre**
  **London WC2E 9RA (GB)**

(54) **A diagnostic method**

(57) A method of detecting the presence of lymphocytes reactive with an antigen in an individual comprising contacting a lymphocyte sample containing T cells and/or B cells from the individual with an agent that non-specifically expands and/or activates a T cell and/or B cell population and said antigen, in order to generate a stimulated lymphocyte population. The detection of an indicator of T cell and/or B cell binding to said antigen in the stimulated lymphocyte population is indicative of the presence of lymphocytes reactive with the antigen being present in the individual.

Figure 5

**Description**

Field of Invention

The present invention relates to a method of detecting the presence of lymphocytes reactive with an antigen in an individual. The present invention also relates to a kit for detecting the presence of lymphocytes reactive with an antigen in an individual.

Background of the Invention

A key function of the adaptive immune response is to protect the body from invading pathogens. Organisms, such as bacteria, viruses and parasites, which infect the body, comprise antigens that are capable of eliciting an adaptive immune response. The adaptive immune response comprises B lymphocytes and T lymphocytes, also termed B cells and T cells, respectively. B cells and T cells have cell surface receptors, termed the B cell receptor and T cell receptor, respectively. The B cell receptor and the T cell receptor bind specifically to a particular site on the antigen, termed an epitope. The B cell receptor can recognise and bind antigen directly, whereas the T cell receptor only binds antigen that is presented on major histocompatibility complex (MHC) proteins on the surface of other cells. The recognition of an epitope by a B or T cell receptor results in activation and proliferation of that particular B cell or T cell to produce an immune response against the antigen.

The adaptive immune response is highly specific to the antigen that elicited it and provides long-lasting protection in the form of immunological memory. In this context, detecting the presence of lymphocytes reactive with a particular antigen can form the basis of a diagnostic assay for infectious disease such as tuberculosis (hereinafter "TB") that indicates whether or not an individual is infected with, or has been exposed to, a pathogen comprising that antigen.

TB is a serious infectious disease caused by certain strains of mycobacteria, principally *Mycobacterium tuberculosis.* It is estimated that one third of the world's population is infected with *M. tuberculosis.* The prevalence of TB varies significantly from country to country. Broadly speaking, TB infection rates in developed countries are relatively low (e.g. in the United Kingdom, the national average was 14.4 cases per 100,000 people in 2012) whereas rates of infection in developing countries are much higher (e.g. 1320 cases per 100,000 people in Swaziland in 2012). Accurate and timely diagnosis of TB infection in individuals and proper management of cases is essential for the effective control of TB.

In relation to diagnostic tests, sensitivity (the true positive rate) can be defined as the proportion of people with disease who will have a positive result, whereas specificity (the true negative rate) can be defined as the proportion of people without the disease who will have a negative result. An ideal diagnostic test would be one with both high sensitivity (i.e. having a low false negative rate) and high specificity (i.e. having a low false positive rate).

There are a range of different techniques that can be used to diagnose TB infection. These include Ziehl-Neelsen staining for acid fast bacilli (AFB), chest X-ray, TB culture and PCR. However, most of these techniques are either low in sensitivity, lack specificity, are expensive and/or require highly trained personnel. For example, TB culture requires biosafety level 3 (BSL-3) laboratory facilities and takes 4 to 8 weeks to obtain results. Therefore, most of these techniques do not fulfil the criteria for point-of-care diagnosis, which is important for the control of TB.

An alternative option for the diagnosis of TB is provided by immune-based assays, which in general exhibit high specificity. One such commercially available assay is the QuantiFERON-TB Gold In-Tube (QFT-GIT) assay. The QFT-GIT assay is a whole blood assay that uses the ESAT-6, CFP-10 and TB 7.7 antigens of *M. tuberculosis.* Incorporation of these antigens into the assay provides greater than 97% specificity. In the QFT-GIT assay, a sample of blood from a subject is contacted with each of three tubes (nil, antigen only and PHA only). PHA is a mitogen and is used as a positive control. The three tubes are incubated at 37°C for 16 to 24 hours, centrifuged and the supernatant is analysed using ELISA to detect cytokine production (IFN-$\gamma$) by T cells in the samples.

However, in contrast to its high specificity, the sensitivity of the QFT-GIT assay is variable. For example, the QFT-GIT assay has been tested in high, medium and low TB endemic countries and the sensitivity of the assay has been found to be lowest in high TB endemic regions, where the need for such a diagnostic assay is extremely high. A number of different reasons have been suggested for the lower sensitivity of the QFT-GIT assay in high TB endemic areas. These reasons include (a) TB patients in low income countries visit health facilities very late when the immune response is impaired and the immune cells have become anergic or exhausted; (b) there is a difference in the recognition of the antigens in the QFT-GIT assay by the T cells of an individual; and (c) there is a difference in the mycobacterium strains (different spoligotypes have been documented) circulating in the different geographical zones so the antigens used in the assay do not exactly correspond to those in the mycobacterium strains to which patients have been exposed. Although these factors can party explain the low sensitivity of the QFT-GIT assay, it is recognised that a range of other factors, such as age of the individual and HIV co-infection can also contribute to the reduced sensitivity of the QFT-GIT assay.

In addition, the applicability of the QFT-GIT assay is contraindicated in various conditions where the subject is immunocompromised, for example, in HIV co-infection, pediatrics, helminthic co-infection and in pregnant women. TB and HIV co-infection is a major health concern, and co-infection is particularly high in sub-Saharan Africa. TB infection is a major cause of death among people living with HIV/AIDS. Therefore, improved diagnosis and management of these two conditions is of great importance. The underlying mechanism of the cellular response to antigens and how cells from different groups of subjects behave is not fully understood. It is therefore difficult to determine which groups of subjects are most likely to be suitable or unsuitable for diagnostic testing using stimulation-based *in vitro* assays, and there is a risk that such assays may produce less than optimal results in certain subject groups. Therefore, there is a need to provide stimulation-based *in vitro* diagnostic assays with improved sensitivity, and, in particular, assays which can be used effectively in subjects that are immunocompromised, and/or which are user-friendly and inexpensive.

The following studies have addressed the limitations of immune-based diagnostic assays. WO 2010/009494 A1 relates to an immune cell-mediated based assay with enhanced sensitivity. The enhanced sensitivity is achieved by providing an agent that, "modulates T cell and in particular T-reg cell activity or function" (page 3, lines 9 to 10, of WO 2010/009494 A1). T regulatory cells are potent suppressors of the inflammatory response and their activity results in a down-regulation of IFN-$\gamma$ production by certain subpopulations of T cells. WO 2010/009494 A1 proposes the use of agents which inhibit regulatory T cell function in order to increase the production of immune effector molecules such as IFN-$\gamma$ by T cells in response to antigen stimulation. Examples of such agents include CD25 ligands and sense or antisense oligonucleotides to particular genes or mRNA encoding molecules such as Janus Kinase 1 (JAKI) or Tyrosine Kinase 2 (TYK2). In addition, WO 2010/009494 A1 proposes the use of stimulating agents such as CpG containing oligonucleotides which act via toll-like receptors (TLRs).

CD25 is a protein expressed on the surface of T regulatory cells. WO 2010/009494 A1 describes the use of anti-CD25 antibody to suppress regulatory T cell function and increase IFN-$\gamma$ production following stimulation with TB antigen in a whole blood sample from one individual. However, the approach of targeting CD25+ T regulatory cells has been shown to produce inconsistent results. For example, a study by Bobosha *et al.* (2014) PLoS Negl Trop Dis. in leprosy patients demonstrated that the depletion of CD25+ T regulatory cells had no effect on IFN-$\gamma$ responses to *Mycobacterium leprae* in 61.1% of patients. In addition, WO 2010/009494 A1 describes the use of antisense oligonucleotides against genetic material encoding JAKI and TYK2. JAKI and TYK2 are involved in the transduction of signalling molecules from regulatory T cells, such as IL-10, which result in suppression of the inflammatory response. Inhibition of the JAKI and TYK2 kinases by antisense oligonucleotides resulted in an increase in IFN-$\gamma$ production following stimulation with TB antigen in a whole blood sample from one individual.

The use of CpG-containing oligonucleotides which act via TLRs was also demonstrated to increase the IFN-$\gamma$ response to TB antigen in a whole blood sample from one individual in WO 2010/009494 A1. TLRs recognise pathogen-associated molecular patterns (PAMPs) and form part of the innate immune system. Activation of the innate immune system can result in indirect activation of cells of the adaptive immune response such as T cells. In addition, there are some reports that certain T cells may express TLRs.

Although WO 2010/009494 A1 refers to modulating "T cell and in particular T-reg cell activity or function", the agents described in the Examples of WO 2010/009494 A1 would not be expected to affect the function or activity of T cells *in general.* Instead, anti-CD25 antibody and antisense oligonucleotides against genetic material encoding JAKI and TYK2 modulate the activity or function of *T regulatory cells.* Furthermore, the use of TLR agonists is only expected to be effective against those sub-populations of T cells that express the appropriate receptor.

However, such assays still result in a sub-optimal level of false positive results. Furthermore, each of the three approaches described in WO 2010/009494 A1 requires molecules which would significantly increase the cost and complexity of the QFT-GIT assay. In addition, WO 2010/009494 A1 describes the use of flow cytometry and/or genetic assays such as PCR to detect immune effector molecules. This would reduce the suitability of the assay for use in developing countries, where suitable funding, laboratory equipment and sufficiently trained personnel may not be available.

Guar et al. (2012) PLOS One. investigated *in vitro* immunomodulation of the QFT-GIT assay using the TLR agonists polyinosine-polycytidylic acid (poly(I:C)), lipopolysaccharide (LPS), and imiquimod (IMQ). The TLR agonists were added to the TB Antigen tube of the QFT-GIT assay to examine whether or not the sensitivity of the assay could be increased. This approach was based on non-specific activation of the innate immune response via the TLR agonists and subsequent indirect activation of T cells. Guar *et al.* demonstrated that modulation of the QFT-GIT assay with TLR agonists enhanced the IFN-$\gamma$ response in subjects with latent tuberculosis infection.

However, the approach of Guar *et al.* was not investigated in immunocompromised individuals and therefore it remains uncertain whether or not the use of TLR agonists would increase the sensitivity of the QFT-GIT assay in this important group of individuals. The addition of LPS at higher concentrations (10 ng/ml) elicited a non-specific response in T cells from uninfected controls. Therefore, there is a concern that this approach may compromise the specificity of the QFT-GIT assay. Furthermore, the magnitude of IFN-$\gamma$ response enhancement varied when immunomodulation was repeated in the same individual on two different occasions. It is recognised that there is differential expression of TLRs on target cells and that diverse conditions contribute to TLR activation *in vivo* in an individual, which cannot be controlled in an *in vitro* assay. Therefore, there is a concern that immunomodulation using TLR agonists will produce inconsistent results between individuals and within a single individual.

There are reports that some T cells may express TLRs. However, the expression of TLRs on T cells is not constitutive and depends on the T cell's functional and activation status. A study by Cottalorda et al. (2009) Eur. J. Immunol. demonstrated that TLR agonists were not sufficient to activate T cells alone and instead required the presence of cytokines, such as IL-2 and IL-7. Therefore, there is a concern that using TLRs to target T cells directly will produce inconsistent and ineffective results because only certain subsets of the T cell population will express TLRs specific to that agonist at a given time point and additional factors are required to ensure robust activation of the T cells.

Aabye et al. (2011) Clin Vaccine Immunol. demonstrated that the cell-mediated immune response to antigen can be enhanced *in vitro* by increasing the incubation temperature from 37°C to 39°C. The effect was further augmented by the addition of the T cell survival cytokine IL-7, which functions by reducing T cell apoptosis and stabilising IFN-γ production, and neutralising antibodies against the anti-inflammatory cytokine IL-10. It was proposed that incubation at 39°C may increase the sensitivity of *in vitro* cell-mediated immune response assays for TB diagnosis, such as the QFT-GIT assay.

However, one drawback of this approach is that the addition of IL-7 and anti-IL-10 monoclonal antibodies, together with an increase in incubation temperature compromised the specificity of the QFT-GIT assay. In addition, a drawback of using IL-7 is that only a subset of T cells express the IL-7 receptor and respond to this cytokine. For example, effector T cells, which produce abundant levels of IFN-γ do not respond to IL-7. This may cause inconsistent results in the QFT-GIT assay as the effect of the IL-7 will depend on the subset of T cells present in the individual's sample. Therefore, there is a need to employ an agent that non-specifically expands and/or activates T cells, regardless of their cell surface receptors, in order to ensure consistent results between individuals and within a single individual.

While the above discussion has focused on the detection of TB, it is also known that the detection of a particular antigen in an individual is useful to diagnose infectious diseases other than TB, such as leprosy, HIV/AIDS, leishmaniasis, malaria, viral hepatitis or meningitis. Alternatively, the detection of an antigen in an individual is associated with cancer and can be used to diagnose cancer in that individual. For example, the antigen may be the product of an oncogene that is associated with cancer in an individual. Thus the present invention is not limited to antigens associated with the presence or development of TB in an individual.

The present invention seeks to alleviate one or more of the above problems. More specifically, the present invention seeks to provide products and methods which improve the sensitivity of stimulation-based *in vitro* diagnostic assays and/or which improve the detection of lymphocytes reactive with an antigen by way of cytokines or antibodies in individuals who are immunocompromised. The products and methods of the present invention arise from the surprising finding that the use of an agent to non-specifically expand and/or activate a T cell population in a sample obtained from an individual can improve the sensitivity of existing stimulation-based *in vitro* diagnostic assays such as the QFT-GIT assay. The products and methods of the present invention provide a simple means of increasing the sensitivity of stimulation-based *in vitro* diagnostic assays and/or improving the detection of antigen in individuals who are immunocompromised.

The present invention can be used as a confirmatory test in eradication programmes to identify all possible cases of a given disease, in blood bank and blood transfusion centres, for early diagnosis of, for example, HIV during the first six months of infection, where the antibody titre in infected individuals is low, as well as for screening purposes at embassies or asylum centres (immigration) where increased sensitivity is required. The test is particularly useful in cases such as TB/HIV-co-infection, pregnant women, pediatrics, helminthic infections, diabetes, TB/cancer co-morbidity, organ transplant and similar conditions.

## Summary of the Invention

In accordance with a first aspect of the invention, there is provided a method of detecting the presence of lymphocytes reactive with an antigen in an individual, comprising the steps of:

    (a) contacting a first lymphocyte sample containing T cells and/or B cells from the individual with: an agent that non-specifically expands and/or activates a T cell and/or a B cell population; and said antigen, in order to generate a first stimulated lymphocyte population; and
    (b) detecting in the first stimulated lymphocyte population an indicator of T cell and/or B cell binding to said antigen, wherein the detection of that indicator is indicative of the presence of lymphocytes reactive with the antigen being present in the individual.

Preferably, step (a) further comprises the step of contacting a second lymphocyte sample containing T cells and/or B cells from the individual with the agent in order to generate a second stimulated lymphocyte population and wherein step (b) further comprises the step of detecting in the second stimulated lymphocyte population the indicator of T cell and/or B cell binding to the antigen, and wherein an increase in the level of the indicator in the first stimulated lymphocyte population relative to the second stimulated lymphocyte population is indicative of the presence of lymphocytes reactive with the antigen being present in the individual.

Conveniently, the detection of the presence of lymphocytes reactive with the antigen is indicative of one of the following pathologies in an individual: tuberculosis, leprosy, HIV/AIDS, leishmaniasis, malaria, viral hepatitis, meningitis or cancer. That is to say, the antigen is associated with the respective pathology.

Advantageously, optical density is used to detect the level of the indicator in the first and/or the second stimulated lymphocyte populations.

Preferably, a ratio of the optical density of the indicator of the first to the second stimulated lymphocyte population is used to determine the presence of lymphocytes reactive with the antigen in the individual, and wherein a ratio greater than 1 is indicative of the presence of lymphocytes reactive with the antigen being present in the individual.

Conveniently, a ratio greater than 1 + k is indicative of the presence of lymphocytes reactive with the antigen being present in the individual, and wherein the value of k is 0.092.

Advantageously, the indicator of T cell binding to the antigen is the production of a cytokine or the production of a mixture of cytokines, preferably wherein the cytokine or one of the cytokines is IFN-γ.

Alternatively, the indicator of B cell binding to the antigen is the production of an immunoglobulin or the production of a mixture of immunoglobulins, preferably wherein the immunoglobulin or the immunoglobulins are selected from IgM, IgG, IgA or IgE.

Preferably, the agent is a mitogen.

Conveniently, the mitogen is phytohaemagglutinin.

Preferably, the individual is in an immunocompromised condition or is a child.

In accordance with a second aspect of the invention, there is provided a kit for detecting the presence of lymphocytes reactive with an antigen in a sample from an individual, comprising:

 (a) an agent that non-specifically expands and/or activates a T cell and/or a B cell population; and
 (b) said antigen.

Preferably, the agent is a mitogen.

Conveniently, the mitogen is phytohaemagglutinin.

Preferably, the antigen is any one of, or a cocktail comprising, ESAT-6, CFP-10 and TB 7.7, and the detection of the presence of lymphocytes reactive with said antigens is indicative of tuberculosis in the individual.

The term "tuberculosis" as used herein refers to any of the infectious diseases of humans or animals caused by species of *Mycobacterium* and characterised by the formation of tubercles and caseous necrosis in the tissues. In some embodiments, the species are *M. tuberculosis* and *M. bovis.*

The term "leprosy" as used herein refers to an infectious disease of humans and certain animals, caused by *Mycobacterium leprae* and characterised by the development of granulomatous or neurotrophic lesions in the skin, mucous membranes, nerves, bones and viscera.

The term "HIV/AIDS" as used herein refers to human immunodeficiency virus infection/acquired immunodeficiency syndrome, which is a transmissible retroviral disease of humans caused by infection with human immunodeficiency virus (HIV). In certain embodiments, HIV/AIDS causes severe depression of cell-mediated immunity.

The term "leishmaniasis" as used herein refers to a disease of humans and animals caused by infection with protozoan parasites of the genus *Leishmania.* The disease is classified into cutaneous leishmaniasis, mucocutaneous leishmaniasis and visceral leishmaniasis.

The term "malaria" as used herein refers to an infectious disease of humans and animals caused by obligate intracellular protozoa of the genus *Plasmodium.* In some embodiments, the disease is transmitted by the bite of infected anopheline mosquitoes.

The term "viral hepatitis" refers to inflammation of the liver due to infection by one of the following viruses: hepatitis A virus, hepatitis B virus, hepatitis C virus, hepatitis D virus and hepatitis E virus.

The term "meningitis" refers to inflammation of the meninges in humans and animals. In certain embodiments, the meningitis is viral meningitis and is caused by a viral infection. Examples of such viruses include the coxsackieviruses, the mumps virus or the lymphocytic choriomeningitis virus. In other embodiments, the meningitis is bacterial meningitis and is caused by a bacterial infection. Examples of such bacteria include *Haemophilus influenza, Neisseria meningitides, Streptococcus pneumonia* or *Mycobacterium tuberculosis.*

The term "cancer" as used herein refers to a group of diseases of humans and animals that are characterised by the abnormal and/or uncontrolled proliferation of cells. Cancer cells have the capacity to invade adjacent tissues and/or to spread to other sites in the body.

The term "lymphocyte" as used herein refers to any of the mononuclear, non-phagocytic leukocytes found in the blood, lymph and lymphoid tissues. The term "lymphocyte" comprises both B lymphocytes and T lymphocytes, which are also referred to as B cells and T cells, respectively.

The term "T-cell" as used herein refers to a cell of the immune system which has a cell surface T-cell receptor and which expresses either the CD8 or the CD4 glycoprotein on its cell surface. The term "T cell" comprises different types of T cell, such as: T helper cells, cytotoxic T cells, memory T cells and regulatory T cells.

The term "B cell" as used herein refers to a cell of the immune system which has a cell surface B-cell receptor. The term "B cell" comprises different types of B cell, such as: plasma B cells, memory B cells, B1 cells and regulatory B cells.

The phrase "an agent that non-specifically expands and/or activates a T cell population" as used herein refers to a chemical or biological entity that has the same effect on all types of T cell and increases the number of T cells in a sample and/or activates the T cell without bias as to the antigen-specificity of the individual T cells. In some embodiments, the term "activates" refers to direct activation of the T cell, without the involvement of other immune cells (i.e. activation that occurs even in the absence of other immune cells). In some embodiments, the term "activate" refers to a mechanism that results in the release of IFN-$\gamma$ from the T cell. In certain embodiments, the optical density (OD) of a secondary reagent is used to determine the relative level of IFN-$\gamma$ as part of the ELISA procedure.

In some embodiments, the agent that non-specifically expands and/or activates a T cell population causes at least a 100% increase in the level of IFN-$\gamma$ in a sample in which T cells from a non-immunocompromised individual are contacted with the agent compared to a sample in which T cells from that individual are not contacted with the agent. In other embodiments, the increase is at least 250%, at least 500%, at least 1,000%, at least 2,000% or at least 5,000%. The concentration of the agent is that used in step (a) of the method of the invention.

Alternatively, in some embodiments, the ratio of the OD of a sample in which T cells from a non-immunocompromised individual are contacted with the agent to a sample in which T cells from that individual are not contacted with the agent is measured. In some embodiments, the ratio of the OD is at least 2. In other embodiments, the ratio is at least 3.5, at least 6, at least 10, at least 20 or at least 50.

The phrase "an agent that non-specifically expands and/or activates a B cell population" as used herein refers to a chemical or biological entity that increases the number of B cells in a sample and/or activates the B cell without bias as to the antigen-specificity of the individual B cells. In some embodiments, the term "activates" refers to direct activation of the B cell, without the involvement of other immune cells (i.e. activation that occurs even in the absence of other immune cells). In some embodiments, the term "activate" refers to a mechanism that results in the production of immunoglobulins by the B cell.

In some embodiments, the agent that non-specifically expands and/or activates a B cell population causes at least a 100% increase in the level of an immunoglobulin in a sample in which B cells from a non-immunocompromised individual are contacted with the agent compared to a sample in which B cells from that individual are not contacted with the agent. In other embodiments, the increase is at least 250%, at least 500%, at least 1,000%, at least 2,000% or at least 5,000%. The concentration of the agent is that used in step (a) of the method of the invention.

The phrase "a stimulated lymphocyte population" as used herein refers to a T cell and/or B cell population which has been non-specifically expanded and/or has been activated.

The term "mitogen" as used herein refers to a substance that triggers a cell to undergo mitosis. In some embodiments, it also refers to lymphocyte blastogenesis.

The phrase "an indicator of T cell binding" as used herein refers to the detection of an indirect or direct outcome of a T cell binding to an antigen. Examples of suitable indicators of T cell binding include the production of cytokines (IL-2, IL-4, IL-5, IL-6, IL-10, IL-17, TNF-$\alpha$, TGF-$\beta$, IFN-$\alpha$, IFN-$\gamma$); chemokines (IP-10, MMP-9, EGF, MCP-3 CX3CL-1); and soluble surface molecules such as sTNFR-I, sTNFR-II. Alternatively, labelled, multimeric complexes of peptide-MHC can be used to directly identify antigen-specific T cells.

The phrase "an indicator of B cell binding" as used herein refers to the detection of an indirect or direct outcome of a B cell binding to an antigen. A suitable indicator of B cell binding is the production of immunoglobulins (IgM, IgG, IgA, IgE).

The term "cytokine" as used herein refers to a cell signalling molecule that is capable of modulating the immune system response. Examples of cytokines comprise chemokines, interferons, interleukins, lymphokines and tumour necrosis factor.

The term "immunocompromised" as used herein refers to an individual whose immune system is not fully functioning or is absent and therefore has a reduced ability to fight infectious diseases. Examples of immunocompromised individuals include: individuals being treated with certain medications (such as chemotherapy and immunosuppressive drugs), individuals suffering from certain types of cancer, HIV infected individuals, helminthic infected individuals, diabetic patients and pregnant women.

Brief Description of the Figures

Figure 1 is a schematic diagram of an assay for the detection of tuberculosis in a sample from an individual in accordance with one embodiment of the present invention.

Figure 2 is a graph of the ELISA results of a proof of concept experiment showing IFN-γ levels under different conditions and additionally showing the delta and normalised OD values in a sample from a TB/HIV co-infected individual (sample 200).

Figure 3 is a graph of the ELISA results from culture supernatant diluted up to 1000 fold showing IFN-γ levels under different conditions in a sample from a healthy tuberculosis skin test (TST)-negative and QFT-negative individual (sample AHRI-01).

Figure 4 is a graph of the ELISA results from culture supernatant diluted up to 1000 fold showing IFN-γ levels under different conditions in a sample from a TB/HIV co-infected individual (sample 200).

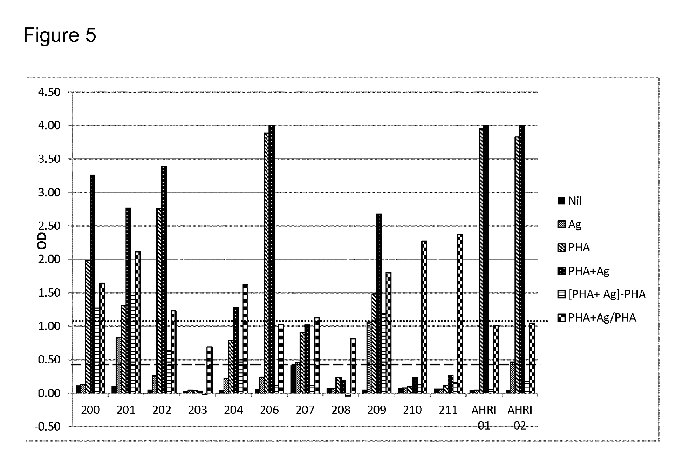

Figure 5 is a graph of the ELISA results showing IFN-γ levels under different conditions and additionally showing the delta and normalised OD values in samples from 9 TB/HIV co-infected individuals (samples 200, 202 to 204 and 206 to 210), 2 TB-infected/HIV negative-individuals (samples 201 and 211), a healthy tuberculosis skin test (TST)-negative and QFT-negative individual (sample AHRI-01) and an apparently healthy (AHRI-02) individual. The dotted line represents the cut off value of 1 + k for the normalised OD approach, where k has the value of 0.092. The dashed line represents the cut off value of 0 + m for the delta OD approach, where m has the value of 0.4.

Figure 6 is a graph of the ELISA results from culture supernatant diluted up to 1000 fold showing IFN-γ levels under different conditions and additionally showing a comparison of delta and normalised OD values in a sample from a TB/HIV co-infected individual (sample 200).

Figure 7 is a graph of the mean ELISA results from culture supernatants diluted up to 100 fold showing IFN-γ levels under different conditions in samples from 10 HIV-negative/TB non-exposed Norwegian individuals.

Figure 8 is a graph of the ELISA results showing IFN-γ levels under different conditions and additionally showing the delta and normalised OD values in samples from 10 HIV-negative/TB non-exposed Norwegian individuals. The dotted line represents the cut off value of 1 + k for the normalised OD approach, where k has the value of 0.092. The dashed line represents the cut off value of 0 + m for the delta OD approach, where m has the value of 0.4.

Detailed Description of the Invention

The present invention is based on the finding that the use of an agent to non-specifically expand and/or activate a T cell population in a sample obtained from an individual improves the sensitivity of existing stimulation-based *in vitro* diagnostic assays such as the QFT-GIT assay. Embodiments of the present invention therefore provide a simple and cost-effective means of increasing the sensitivity of TB diagnosis and/or improving the detection of TB in individuals who are immunocompromised.

In one embodiment, a kit is provided for the detection of tuberculosis in an individual. The kit comprises: a supply of phytohaemagglutinin (PHA); supplies of the following antigens of M. *tuberculosis:* ESAT-6, CFP-10 and TB 7.7; and four vials.

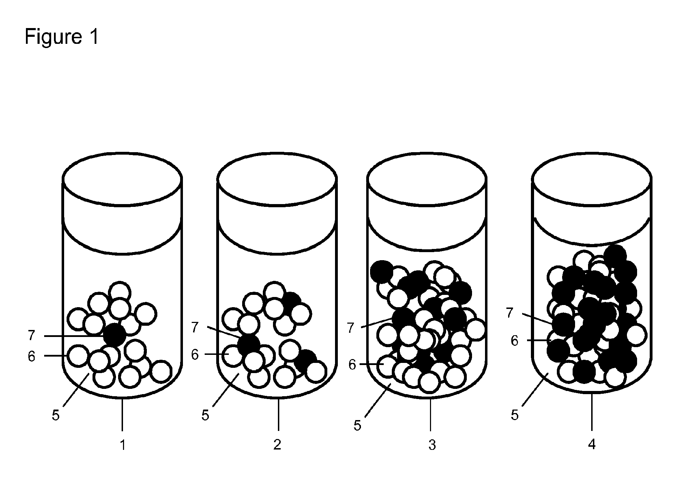

Referring to Figure 1, use of the kit in accordance with a first embodiment will now be described. In use of the kit, a blood sample is taken from an individual. The individual to be tested may have symptoms which suggest possible infection with TB. Alternatively, the individual may be tested because he or she has had contact with another individual who has been infected with TB or may present as part of a general screening programme. The individual may be in an immunocompromised condition or may be a child. A portion of the blood sample provided by the individual is added to each of the four vials 1 to 4. It is preferred that the vials are blood collection tubes. Referring to Figure 1, the blood sample 5 contains a population of T cells from the individual, a first fraction 6 of which are not reactive with the TB antigens provided and a second fraction 7 of which are reactive with the TB antigens provided.

Referring, still, to Figure 1, once the blood sample has been added to each of the vials 1 to 4, nothing further is added to the first vial 1. With regard to the other three vials 2 to 4, the following components of the kit are added: the ESAT-6, CFP-10 and TB 7.7 antigens of M. *tuberculosis* are added to the second vial 2; the PHA is added to the third vial 3; and the ESAT-6, CFP-10 and TB 7.7 antigens of M. *tuberculosis* and the PHA are both added to the fourth vial 4.

In the first vial 1, there is no change in the number of T cells of either the first or second fraction (6 and 7) in the blood sample from the individual.

In the second vial 2, which contains the ESAT-6, CFP-10 and TB 7.7 antigens of M. *tuberculosis,* there is an increase in the number of T cells of the second fraction 7 in the blood sample, i.e. T cells which are reactive with these antigens.

In the third vial 3, which contains the PHA, the PHA results in an overall increase in the number of T cells of the first and second fractions (6 and 7) in the blood sample, i.e. without bias as to the antigen-specificity of the individual T cells.

In the fourth vial 4, which contains the ESAT-6, CFP-10 and TB 7.7 antigens of M. *tuberculosis* and the PHA, the PHA results in an overall increase in the number of T cells of the first and second fractions (6 and 7). In addition, the ESAT-6, CFP-10 and TB 7.7 antigens cause a specific increase in the number of T cells of the second fraction 7 in the blood sample, i.e. T cells which are reactive with these antigens. Therefore, the number of antigen-specific T cells of the second fraction 7 is highest in the fourth vial 4 and is proportionally higher than the number of T cells of the first fraction than in any of the first to third vials (1 to 3).

In the first embodiment, Enzyme-Linked Immunosorbent Assay (ELISA) is used to detect the level of the cytokine IFN-$\gamma$ in each of the four vials. IFN-$\gamma$ is released by the T cells in the blood sample and in particular by those T cells which have bound to one of the ESAT-6, CFP-10 and TB 7.7 antigens of M. *tuberculosis.* Therefore, the level of IFN-$\gamma$ in the blood sample is used as an indicator of T cell binding to the ESAT-6, CFP-10 and TB 7.7 antigens. The optical density (OD) of a secondary reagent is used to determine the relative level of IFN-$\gamma$ as part of the ELISA procedure. In alternative embodiments, the OD is then used to calculate the concentration of IFN-$\gamma$.

However, in other embodiments, the production of cytokines other than IFN-$\gamma$ is used as the indicator of T cell binding to the at least one antigen of M. *tuberculosis.* In one embodiment, a technique such as ELISA is used to detect the level of the cytokines produced by the T cells. In a further embodiment, the use of labelled, multimeric complexes of peptide-MHC is used to identify antigen-specific T cells directly.

Referring to the first embodiment, the level of IFN-$\gamma$, as determined by OD, in the fourth vial (containing the PHA and the ESAT-6, CFP-10 and TB 7.7 antigens of M. *tuberculosis)* is compared with the level of IFN-$\gamma$, as determined by OD, in the third vial (containing the PHA alone) in order to calculate a delta or normalised OD value as follows:

$$\text{Delta OD} = \text{OD of the fourth vial (PHA + antigens)} - \text{OD of third vial (PHA)}$$
$$= \text{OD (antigen amplified)}$$

Or

$$\text{Normalised OD} = \text{OD of the fourth vial (PHA + antigens)/OD of third vial (PHA)}$$

In certain embodiments, it is preferable to set a cut off value using the normalised OD approach because this approach accounts for differences in the magnitude of an individual's blood sample's response to the PHA and/or the antigen. The magnitude of IFN-$\gamma$ released from a T cell that is contacted with PHA and/or the antigen will be affected by a number of factors including the individual from whom the T cells were obtained and their health status. Therefore, in certain embodiments, the use of a cut off value and the normalised OD approach improves the sensitivity of the method in individuals whose T cells do not produce large overall amounts of IFN-$\gamma$ in response to PHA and/or the antigen. The expected minimum value of the normalisation (ratio) of OD between the fourth vial and the third vial is 1 because if an individual has not been exposed to the antigens of M. *tuberculosis* previously, the level of IFN-$\gamma$ in the fourth vial and the third vial will be approximately equal. An individual is diagnosed with TB infection if the normalised OD value is greater than 1 + k, where k is the correction factor and has a value of 0.092.

In some embodiments, a cut off value using the delta OD approach is set. The expected minimum value of the delta OD approach is 0 because if an individual has not been exposed to the antigens of M. *tuberculosis* previously, the level of IFN-$\gamma$ in the fourth vial and the third vial will be approximately equal. An individual is diagnosed with TB infection if the delta OD value is greater than 0 + m, where m is the correction factor and has a value of 0.4.

It should be noted that in certain embodiments of the kit, the supplies of PHA and/or the antigens of M. *tuberculosis* are provided in the vials of the kit before the blood sample from the individual is added to the vial. Therefore, in this embodiment, the kit comprises the following four vials: the first vial contains nothing; the second vial contains the ESAT-6, CFP-10 and TB 7.7 antigens of M. *tuberculosis;* the third vial contains PHA; and the fourth vial contains the ESAT-6, CFP-10 and TB 7.7 antigens of *M. tuberculosis* and PHA. In use of the kit, a blood sample is taken from an individual and a portion is added to each of the four vials in order to make a diagnosis of TB infection in the individual.

In the first embodiment, the agent that non-specifically expands and/or activates the T cell population is PHA. However, in another embodiment, the agent that non-specifically expands and/or activates the T cell population is anti-CD3 antibody. In one embodiment, anti-CD3 antibody is used in combination with one or more additional factors to non-specifically expand and/or activate the T cell population. Such factors include cytokines, such as IL-2, and anti-CD28 antibody. In further embodiments, the agent is a mitogen (other than PHA). In one embodiment the agent is a lectin (other than PHA) that is capable of mitogenic stimulation. Examples of such lectins include: *Artocarpus integrifolia* lectin (jacalin), Concanavalin A from *Canavalia ensiformis, Erythrina corallodendron* lectin and *Maackia amurensis* lectin. Alternatively, in further embodiment, the mitogen is a combination of the phorbol ester PMA and the calcium ionophore ionomycin.

In some embodiments, the level of IFN-γ released from the T cell is measured and is used to determine whether a substance is an agent non-specifically expands and/or activates the T cell population. In some embodiments, a substance can be tested to determine if it is suitable as an agent that non-specifically expands and/or activates a T cell population by determining if it causes at least a 100% increase in the level of IFN-γ in a sample in which T cells from a non-immunocompromised individual are contacted with the substance compared to a sample in which T cells from that individual are not contacted with the substance. In other embodiments, the increase is at least 250%, at least 500%, at least 1,000%, at least 2,000% or at least 5,000%. The concentration of the substance is the same as the concentration of the agent that is used in step (a) of the method of the invention (i.e. the concentration at which the agent is added to the fourth vial containing the antigen or antigens).

In the first embodiment, a combination of the ESAT-6, CFP-10 and TB 7.7 antigens of *M. tuberculosis* is provided in the kit. Exemplary accession numbers from the GenBank database for the ESAT-6, CFP-10 and TB 7.7 antigens are FJ014499.1, FJ014498.1 and P9WJX1, respectively. However, in other embodiments, one or a combination of any two of the ESAT-6, CFP-10 or TB 7.7 antigens is provided in the kit. In still further embodiments, other antigens of *M. tuberculosis* are provided in the kit in addition to, or instead of, the ESAT-6, CFP-10 or TB 7.7 antigens.

The principle of the approach described herein is based on *in vitro* stimulation using an agent that non-specifically expands and/or activates a T cell population. While not wishing to be bound by theory, it is believed that use of the agent induces a new pool of T cells thereby enhancing the number of antigen-specific T cells and/or the quality of the antigen-specific T cells to produce new antigen-specific T cells. This approach facilitates the detection of indicators of T cell binding to antigen(s) of *M. tuberculosis,* thereby increasing the sensitivity of the assay whilst maintaining specificity.

In the embodiments described above, the antigen provided in the kit is from *M. tuberculosis* and the kit is used to detect the presence of tuberculosis in an individual. However, in other embodiments, the antigen is instead from, or is associated with infection by, *M. leprae,* HIV, protozoan parasites of the genus *Leishmania,* protozoa of the genus *Plasmodium,* hepatitis A, B, C, D or E virus, or viruses or bacteria capable of causing inflammation of the meninges. Detecting the presence of lymphocytes reactive with said antigens in an individual is indicative of one of the following pathologies in the individual: leprosy, HIV/AIDS, leishmaniasis, malaria, viral hepatitis or meningitis, respectively. In a further embodiment, the kit comprises an antigen that is associated with cancer in an individual and is used to detect the presence of cancer in the individual. In the first such embodiment, the antigen that is associated with cancer is an abnormal product of the P53 gene. In the second such embodiment, the antigen is a Receptor tyrosine-protein kinase erbB-2 peptide.

In the embodiments described above, the kit comprises an agent that non-specifically expands and/or activates a T cell population. However, in another embodiment the kit comprises an agent that non-specifically expands and/or activates a B cell population. In one embodiment, said agent is a mitogen. In another embodiment, the mitogen is a lectin that is capable of mitogenic stimulation. Examples of such lectins are *Erythrina corallodendron* lectin, *Maackia amurensis* lectin, *Phytolacca americana* lectin (pokeweed mitogen). In yet another embodiment of the present invention, the agent supplied in the kit non-specifically expands and/or activates a T cell and a B cell population.

In some embodiments, the level of an immunoglobulin produced by the B cell is measured and is used to determine whether a substance is an agent non-specifically expands and/or activates the B cell population. In some embodiments, a substance can be tested to determine if it is suitable as an agent that non-specifically expands and/or activates a B cell population by determining if it causes at least a 100% increase in the level of an immunoglobulin in a sample in which B cells from a non-immunocompromised individual are contacted with the substance compared to a sample in which B cells from that individual are not contacted with the substance. In other embodiments, the increase is at least 250%, at least 500%, at least 1,000%, at least 2,000% or at least 5,000%. The concentration of the substance is the same as the concentration of the agent that is used in step (a) of the method of the invention (i.e. the concentration at which the agent is added to the fourth vial containing the antigen or antigens).

In the embodiments in which the kit comprises an agent that non-specifically expands and/or activates a B cell population, an indicator of B cell binding is used to detect an indirect or direct outcome of a B cell binding to an antigen. A suitable indicator of B cell binding is the production of immunoglobulins (IgM, IgG, IgA, IgE).

<u>Examples</u>

<u>Materials and Methods</u>

4 millilitres (ml) of heparinized peripheral blood was collected from each subject and 1 ml of fresh whole blood was added into each of the three QFT tubes (NIL, Antigen and Mitogen) and mixed by inverting 10 times while 1 ml was added into another Antigen tube, mixed by inverting 10 times to wash off all the antigen on the tube surface, transferred into another Mitogen tube and mixed by inverting 10 times. All the 4 tubes were incubated at 37°C overnight. After 20 hours the supernatant was collected by centrifuging the tubes at 3000 rpm for 10 minutes. The IFN-γ level was determined by ELISA using the following protocol, which is provided in the QFT-GIT assay.

<u>IFN-γ ELISA protocol</u>

1. All plasma samples and reagents, except for Conjugate 100X Concentrate, must be brought to room temperature (22°C ± 5°C) before use. Allow at least 60 minutes for equilibration.
2. Reconstitute freeze dried Conjugate 100X Concentrate with 0.3mL of deionised or distilled water. Mix gently to minimize frothing and ensure complete solubilisation of the Conjugate. Working Strength conjugate is prepared by diluting the required amount of reconstituted Conjugate 100X Concentrate in Green Diluent as set out in Table 1 - Conjugate Preparation.
3. Prior to assay, plasmas should be mixed to ensure that IFN-γ is evenly distributed throughout the sample. Plasma should be in a 96 well plate layout.
4. Add 50μL of freshly prepared Working Strength conjugate to the required ELISA wells using a multichannel pipette.
5. Add 50μL of test plasma samples to appropriate wells using a multichannel.
6. Mix the conjugate and plasma samples thoroughly using a microplate shaker for 1 minute.
7. Cover each plate with a lid and incubate at room temperature (22°C ± 5°C) for 120 ± 5 minutes. Plates should not be exposed to direct sunlight during incubation.
8. During the incubation, dilute one part Wash Buffer 20X Concentrate with 19 parts deionised or distilled water and mix thoroughly. Sufficient Wash Buffer 20X Concentrate has been provided to prepare 2L of Working Strength wash buffer.

- Wash wells with 400μL of Working Strength wash buffer for at least 6 cycles. An automated plate washer is recommended.
- Thorough washing is very important to the performance of the assay. Ensure each well is completely filled with wash buffer to the top of the well for each wash cycle. A soak period of at least 5 seconds between each cycle is recommended.
- Standard laboratory disinfectant should be added to the effluent reservoir, and established procedures followed for the decontamination of potentially infectious material.

9. Tap plates face down on absorbent towel to remove residual wash buffer. Add 100μL of Enzyme Substrate Solution to each well and mix thoroughly using a microplate shaker.
10. Cover each plate with a lid and incubate at room temperature (22°C ± 5°C) for 30 minutes. Plates should not be exposed to direct sunlight during incubation.
11. Following the 30 minute incubation, add 50μL of Enzyme Stopping Solution to each well and mix. Enzyme Stopping Solution should be added to wells in the same order and at approximately the same speed as the substrate in step 9.
12. Measure the Optical Density (OD) of each well within 5 minutes of stopping the reaction using a microplate reader fitted with a 450nm filter and with a 620nm to 650nm reference filter. OD values are used to calculate results.

Subsequent analysis was performed by calculating a delta and/or normalized OD value as follows:

Delta OD = OD of the fourth vial (PHA + antigens) – OD of third vial (PHA)

= OD (antigen amplified)

Or

## Normalised OD = OD of the fourth vial (PHA + antigens)/OD of third vial (PHA)

An individual was diagnosed with TB infection if the delta OD value was greater than the cut off value of 0 + m, where m had a value of 0.4; or if the normalised OD value was greater than 1 + k, where k had a value of 0.092. The value of k was calculated from the average OD value of the Nil tubes of the 13 samples described in the following Examples.

Test subjects

The clinical and demographic characteristics of the subjects used in this study are set out in Table 1.

| Sample | Subject profile | | | | | |
|---|---|---|---|---|---|---|
| | TB | HIV | CD4 | age | sex | WEIGHT |
| 200 | SNPTB | positive | 261 | 38 | M | 52kg |
| 201 | SNPTB | negative | NA | 40 | M | 58kg |
| 202 | SNPTB | positive | 154 | 25 | M | 56kg |
| 203 | SNPTB | positive | 26 | 45 | M | NA |
| 204 | SNPTB | positive | 39 | 38 | M | 51kg |
| 206 | SNPTB | positive | 201 | 52 | M | 50kg |
| 207 | EPTB | positive | 499 | 36 | M | 47kg |
| 208 | SNPTB | positive | 233 | 34 | F | 42kg |
| 209 | SPTB | positive | 140 | 21 | F | 47kg |
| 210 | SNPTB | positive | 79 | 45 | M | 44kg |
| 211 | EPTB | negative | NA | 20 | M | 51kg |
| AHRI 01 | negative | negative | NA | 34 | F | 64kg |
| AHRI 02 | negative | negative | NA | 32 | F | 55kg |

Example 1: Detection of TB in a TB-HIV co-infected individual

The assay was carried out on one TB/HIV co-infected individual as a proof of concept experiment (sample 200). A blood sample was collected from the individual and the assay was carried out as per the materials and methods. The IFN-$\gamma$ level in each tube (Nil, Antigen, Mitogen and Antigen plus Mitogen) was determined by ELISA as per the materials and methods. Delta and normalised OD values were calculated.

**Table 2: IFN-$\gamma$ levels under different conditions in a sample from a TB/HIV co-infected individual**

| PHA+Ag | PHA | Norm OD | Delta OD | Ag | Nil |
|---|---|---|---|---|---|
| 3.26 | 1.99 | 1.64 | 1.27 | 0.13 | 0.11 |

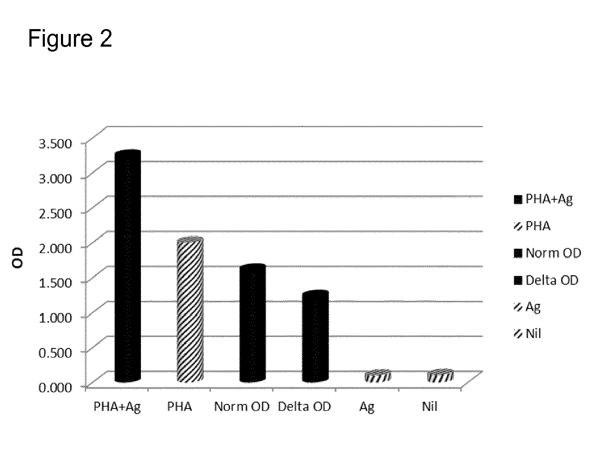

The results from Table 2 are shown graphically in Figure 2. Referring to Table 2 and Figure 2, the IFN-$\gamma$ level in the Mitogen plus Antigen (PHA + Ag) tube was high compared to the IFN-$\gamma$ level in the Antigen (Ag) tube. The normalised OD value of 1.64 was higher than the threshold value of 1 + k i.e. 1.092. Therefore, in this example, the subject was correctly identified as TB positive using the normalised OD approach. Had the prior art QFT-GIT assay been used (i.e. using only Nil, Antigen and Mitogen tubes) the subject might not have been identified as TB positive because the difference between the IFN-$\gamma$ level in the Nil tube and the Ag tube was very small and therefore it might not have passed the cut off value set by the manufacturer for the Ag tube (> or =0.35IU).

In this example, both delta OD and normalised OD values were calculated using the data from the PHA and PHA + Ag tubes. Referring again to Figure 2 and Table 2, there was a notable difference between both the calculated delta OD and the normalised OD values and the IFN-γ level of the Nil tube. Therefore, this example correctly identified the TB/HIV co-infected individual as TB positive using the normalised OD approach.

Example 2: Culture supernatant dilutions demonstrate that there is no non-specific IFN-γ production due to co-stimulation with mitogen and TB antigens

This example was carried out on a healthy tuberculosis skin test (TST)-negative and QFT-negative individual (sample AHRI-01) and a TB/HIV co-infected individual (sample 200). A blood sample was collected from each individual and the example was carried out as per the materials and methods, up until the centrifugation step. Following centrifugation at 3000 rpm for 10 minutes, the supernatant from each of the four tubes from each individual was collected, diluted up to 1000 fold, and the level of IFN-γ was detected using ELISA in an undiluted sample and in each of the diluted samples. This dilution was conducted in case the IFN-γ level had reached a saturated phase, which would make it difficult to ascertain any difference in the level of IFN-γ between the Mitogen (PHA) and Mitogen plus Antigen PHA (PHA + Ag) samples.

Results

**Table 3: IFN-γ levels under different conditions in a sample from a healthy individual**

| Sample AHRI-01 | | Un-diluted | 10 fold | 100 fold | 1000 fold |
|---|---|---|---|---|---|
| | **Nil** | 0.048 | 0.039 | 0.04 | 0.034 |
| | **Ag** | 0.056 | 0.043 | 0.047 | 0.053 |
| | **PHA** | 4 | 4 | 0.915 | 0.161 |
| | **PHA+Ag** | 4 | 3.9 | 0.713 | 0.175 |

**Table 4: IFN-γ levels under different conditions in a sample from a TB/HIV co-infected individual**

| Sample 200 | | Un diluted | 1:1 | 10 fold | 100 fold | 1000 fold |
|---|---|---|---|---|---|---|
| | **Nil** | 0.1085 | 0.046 | 0.03 | 0.047 | 0.031 |
| | **Ag** | 0.125 | 0.032 | 0.032 | 0.032 | 0.031 |
| | **PHA** | 1.98525 | 1.885 | 0.654 | 0.091 | 0.039 |
| | **PHA+Ag** | 3.26 | 3.298 | 1.618 | 0.309 | 0.065 |

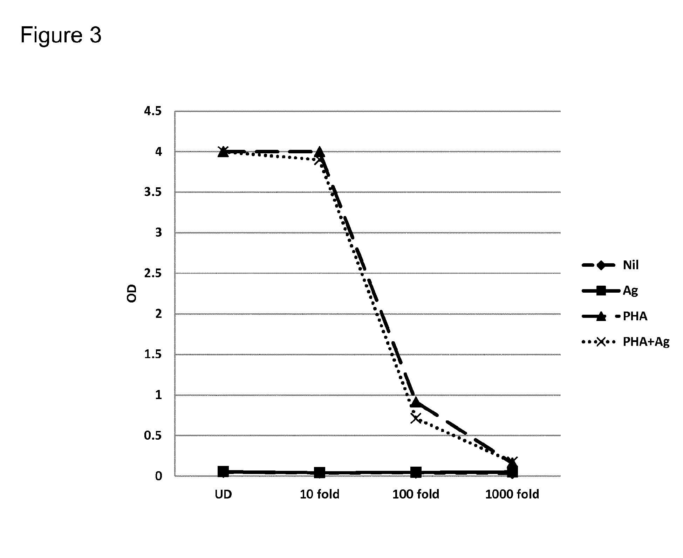

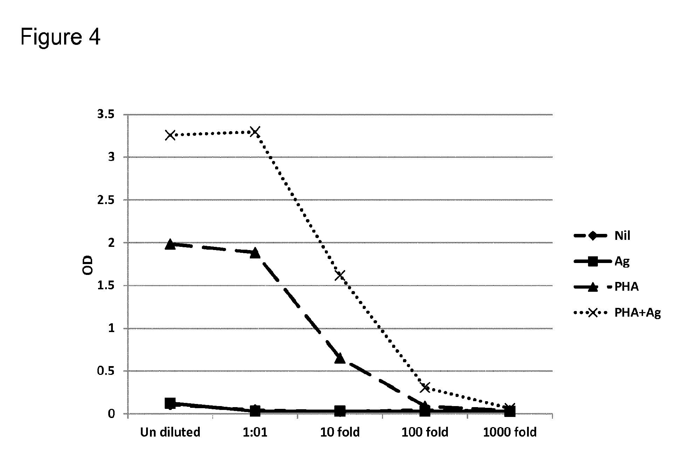

The results from Tables 3 and 4 are shown graphically in Figures 3 and 4, respectively. Referring to Table 3 and Figure 3, which relate to a healthy individual, the IFN-γ level in the Mitogen (PHA) and Mitogen plus Antigen (PHA + Ag) tubes was very similar, irrespective of the dilution used. The IFN-γ level in Antigen (Ag) tube was similar to the Nil tube in the undiluted sample and in each of the diluted samples. The similarity in the level of IFN-γ between the PHA and PHA + Ag tubes in the undiluted sample and in each of the dilutions tested suggests that there was no non-specific IFN-γ production by the T cells of the healthy individual following co-stimulation with PHA and TB antigens.
Referring to Table 4 and Figure 4, which relate to a TB/HIV co-infected individual, the IFN-γ level in the PHA + Ag tube was higher than the IFN-γ level in the PHA tube in the undiluted sample and in each of the diluted samples. The difference in the IFN-γ level between the PHA + Ag and PHA tubes was particularly noticeable in the undiluted sample and in the 1:1 and 10-fold dilutions. In the undiluted sample, the level of IFN-γ was slightly higher in the Ag tube as compared to the Nil tube. However, at the four dilutions tested, the IFN-γ level in the Ag and Nil tubes was generally similar. The level of IFN-γ was lower in the Ag tube as compared to the PHA + Ag tube in the undiluted sample and in each of the dilutions tested. The results indicate that co-stimulation of the T cells of a TB/HIV co-infected individual with PHA and TB antigens resulted in increased antigen-specific IFN-γ production.

Conclusions

Co-stimulation of T cells with a mitogen (PHA) and TB antigens did not result in non-specific IFN-γ production by T cells in a sample from a healthy individual but it did increase IFN-γ production by T cells in a sample from a TB/HIV co-infected individual. Healthy and TB-infected individuals were distinguished in the example.

Example 3: Detection of TB

This example was carried out on 9 TB/HIV co-infected individuals (samples 200, 202 to 204 and 206 to 210), 2 TB-infected/HIV-negative individuals (samples 201 and 211), a healthy tuberculosis skin test (TST)-negative and QFT-negative individual (sample AHRI-01) and an apparently healthy (AHRI-02) individual. A blood sample was collected from each individual and the assay was carried out as per the materials and methods. The IFN-γ level in each tube (Nil, Antigen, Mitogen and Antigen plus Mitogen) was determined by ELISA for each individual as per the materials and methods. Delta and normalised OD values were calculated. An assumed cut off value was calculated in order to determine individuals who were positive for TB infection. The calculated cut off value for the normalised OD approach was 1 + k, where k had the value of 0.092. The calculated cut off value for the delta OD approach was 0 + m, where m had the value of 0.4.

**Table 5: IFN-γ levels under different conditions in samples from 9 TB/HIV co-infected individuals, 2 TB-positive/HIV-negative individuals and 2 healthy individuals**

| Sample | 200 | 201 | 202 | 203 | 204 | 206 | 207 | 208 | 209 | 210 | 211 | AHRI 01 | AHRI 02 |
|---|---|---|---|---|---|---|---|---|---|---|---|---|---|
| Nil | 0.11 | 0.1 | 0.06 | 0.03 | 0.05 | 0.06 | 0.41 | 0.08 | 0.06 | 0.08 | 0.07 | 0.05 | 0.04 |
| Ag | 0.13 | 0.82 | 0.26 | 0.05 | 0.22 | 0.23 | 0.46 | 0.07 | 1.06 | 0.08 | 0.06 | 0.05 | 0.47 |
| PHA | 1.99 | 1.31 | 2.76 | 0.04 | 0.79 | 3.89 | 0.9 | 0.23 | 1.48 | 0.1 | 0.11 | 3.95 | 3.83 |
| PHA+Ag | 3.26 | 2.77 | 3.39 | 0.03 | 1.28 | 4 | 1.02 | 0.19 | 2.67 | 0.23 | 0.26 | 4 | 4 |
| [PHA+ Ag]-PHA | 1.27 | 1.46 | 0.63 | -0.01 | 0.49 | 0.11 | 0.12 | -0.04 | 1.19 | 0.13 | 0.15 | 0.05 | 0.17 |
| PHA+Ag/PHA | 1.64 | 2.11 | 1.23 | 0.69 | 1.63 | 1.03 | 1.13 | 0.81 | 1.81 | 2.27 | 2.37 | 1.01 | 1.04 |

Results

The results from Table 5 are shown graphically in Figure 5. Referring to Figure 5, the results show that, using an assumed cut off value of 1.092 (represented by the dotted line in Figure 5) the normalized OD value detected 6 out of the 9 TB/HIV co-infected TB individuals as being TB positive and detected the 2 TB-positive/HIV-negative individuals as being TB positive.

In addition, using an assumed cut off value of 0.4 (represented by the dashed line in Figure 5) the delta OD value detected 4 out of the 9 TB/HIV co-infected TB individuals as being TB positive and only 1 of the 2 TB-positive/HIV-negative individuals as being TB positive. Therefore, the normalised OD approach detected more cases of TB in the samples used in this example than the delta OD approach.

The two healthy individuals were correctly identified as TB negative by either the delta OD or the normalised OD approach.

Referring to Table 1, it should be noted that among the 9 TB/HIV co-infected individuals, sample 203 had the lowest CD4 count. As shown in Figure 5, the assay did not detect this individual as TB positive. Sample 203 was not detected as TB positive by the QFT-GIT assay. Sample 207 had the highest background IFN-γ response in the Nil tube. This may be a result of disseminated TB disease. While the overall response was low for samples 210 and 211, these individuals were detected as TB positive by the assay using the normalised OD approach.

Conclusions

Using a cut-off value, the normalised OD approach correctly detected more cases of TB in 9 TB/HIV co-infected individuals and 2 TB-positive/HIV-negative individuals than the delta OD approach.

Example 4: Effect of dilution on the magnitude of IFN-γ level

The assay was carried out on a TB/HIV co-infected individual (sample 200). A blood sample was collected from the individual and the assay was carried out as per the materials and methods, up until the centrifugation step. Following centrifugation at 3000 rpm for 10 minutes, the supernatant from each of the four tubes from the individual was collected, diluted up to 1000 fold, and the level of IFN-γ was detected using ELISA in an undiluted sample and in each of the diluted samples. Delta and normalised OD values were calculated for the undiluted sample and for each dilution.

Results

**Table 6: IFN-γ levels under different conditions in a sample from a TB/HIV co-infected individual**

| Sample 200 | | Undiluted | 2 fold | 10 fold | 100 fold | 1000 fold |
|---|---|---|---|---|---|---|
| | Nil | 0.11 | 0.046 | 0.03 | 0.047 | 0.031 |
| | Ag | 0.13 | 0.032 | 0.032 | 0.032 | 0.031 |
| | PHA | 1.99 | 1.885 | 0.654 | 0.091 | 0.039 |
| | PHA+Ag | 3.26 | 3.298 | 1.618 | 0.309 | 0.065 |
| | [PHA+Ag]-PHA | 1.27 | 1.41 | 0.96 | 0.22 | 0.03 |
| | [PHA+Ag]/PHA | 1.64 | 1.75 | 2.47 | 3.4 | 1.67 |

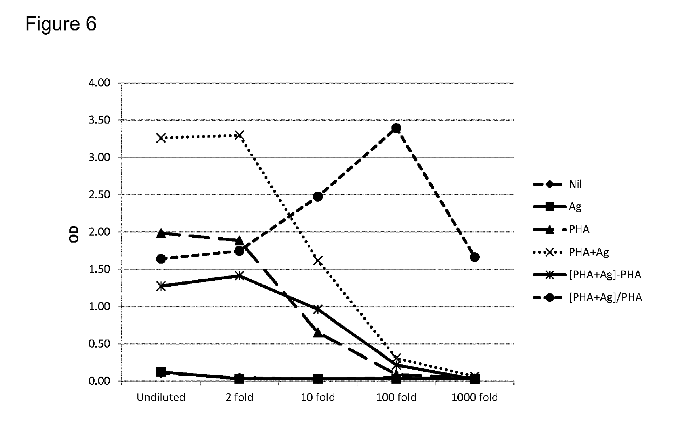

The results from Table 6 are shown graphically in Figure 6. Referring to Figure 6, the calculated normalised OD value was higher than the delta OD value in the undiluted sample and in each of the diluted samples. In this particular example, the highest normalised OD value was measured at 100-fold dilution of the culture supernatant. The higher value of the normalised OD compared to the delta OD suggests that the former value is more effective in determining individuals who are TB positive.

Conclusion

The calculated normalised OD value was higher than the delta OD value in undiluted and diluted samples of the culture supernatant, suggesting that the former value is a superior measure of IFN-γ levels in the assay.

Example 5: No non-specific IFN-γ production is detected in HIV-negative/TB non-exposed Norwegian individuals

The assay was carried out on 10 HIV-negative/TB non-exposed healthy Norwegian individuals (samples NIPH 01 to NIPH 10). A blood sample was collected from each individual and the assay was carried out as per the materials and methods, up until the centrifugation step. Following centrifugation at 3000 rpm for 10 minutes, the supernatant from each of the four tubes from each individual was collected, diluted up to 100 fold, and the level of IFN-γ was detected using ELISA in an undiluted sample and in each of the diluted samples. This experiment was carried out as a negative control to confirm that there is no non-specific IFN-γ production by the T cells of TB non-exposed individuals following co-stimulation with PHA + TB antigens. The advantage of using Norwegian individuals is that these individuals are unlikely to have been exposed to TB as the disease is not endemic in Norway. Therefore, these individuals can be used as negative controls with confidence.

Results

**Table 7: IFN-γ levels under different conditions in samples from 10 HIV-negative/TB non-exposed healthy Norwegian individuals**

| | Nil | | | Ag | | | PHA | | | PHA+Ag | | |
|---|---|---|---|---|---|---|---|---|---|---|---|---|
| Sample | UD | 10fold | 100fold | UD | 10fold | 100fold | UD | 10fold | 100fold | UD | 10fold | 100fold |
| NIPH 01 | 0.042 | 0.039 | 0.067 | 0.099 | 0.048 | 0.068 | 3.957 | 1.32 | 0.066 | 3.753 | 1.203 | 0.198 |
| NIPH 02 | 0.063 | 0.047 | 0.052 | 0.055 | 0.047 | 0.045 | 4.164 | 3.832 | 1.13 | 4.082 | 2.858 | 0.576 |
| NIPH 03 | 0.076 | 0.056 | 0.069 | 0.076 | 0.049 | 0.056 | 4.03 | 2.618 | 0.393 | 3.905 | 1.287 | 0.202 |
| NIPH 04 | 0.06 | 0.054 | 0.052 | 0.064 | 0.043 | 0.048 | 4.03 | 2.74 | 0.467 | 4.017 | 2.439 | 0.422 |
| NIPH 05 | 0.065 | 0.05 | 0.061 | 0.074 | 0.052 | 0.077 | 4.071 | 2.061 | 0.244 | 3.995 | 1.122 | 0.306 |
| NIPH 06 | 0.063 | 0.042 | 0.049 | 0.047 | 0.07 | 0.082 | 4.146 | 3.652 | 0.713 | 4.115 | 3.896 | 0.96 |
| NIPH 07 | 0.082 | 0.06 | 0.067 | 0.182 | 0.084 | 0.065 | 4.139 | 4.061 | 1.287 | 4.109 | 3.317 | 0.554 |
| NIPH 08 | 0.056 | 0.063 | 0.085 | 0.065 | 0.079 | 0.088 | 3.791 | 0.907 | 0.178 | 3.5 | 0.749 | 0.155 |
| NIPH 09 | 0.089 | 0.086 | 0.084 | 0.1 | 0.089 | 0.085 | 4.037 | 1.464 | 0.229 | 4.094 | 1.66 | 0.246 |
| NIPH-10 | 0.109 | 0.051 | 0.064 | 0.088 | 0.068 | 0.056 | 3.953 | 3.711 | 0.729 | 4.054 | 2.97 | 0.435 |
| Mean | 0.0705 | 0.0548 | 0.065 | 0.085 | 0.0629 | 0.067 | 4.0318 | 2.6366 | 0.5436 | 3.9624 | 2.1501 | 0.4054 |

**Table 8: IFN-γ levels under different conditions and delta and normalised OD values in samples from 10 HIV-negative/TB non-exposed healthy Norwegian individuals**

| Sample | Nil | Antigen | PHA | PHA+Ag | [PHA+Ag] - PHA | PHA+Ag/PHA |
|---|---|---|---|---|---|---|
| NIPH 01 | 0.042 | 0.099 | 3.957 | 3.753 | -0.204 | 0.948446 |
| NIPH 02 | 0.063 | 0.055 | 4.164 | 4.082 | -0.082 | 0.980307 |
| NIPH 03 | 0.076 | 0.076 | 4.03 | 3.905 | -0.125 | 0.968983 |
| NIPH 04 | 0.06 | 0.064 | 4.03 | 4.017 | -0.013 | 0.996774 |
| NIPH 05 | 0.065 | 0.074 | 4.071 | 3.995 | -0.076 | 0.981331 |
| NIPH 06 | 0.063 | 0.047 | 4.146 | 4.115 | -0.031 | 0.992523 |
| NIPH 07 | 0.082 | 0.182 | 4.139 | 4.109 | -0.03 | 0.992752 |
| NIPH 08 | 0.056 | 0.065 | 3.791 | 3.5 | -0.291 | 0.923239 |
| NIPH 09 | 0.089 | 0.1 | 4.037 | 4.094 | 0.057 | 1.014119 |
| NIPH-10 | 0.109 | 0.088 | 3.953 | 4.054 | 0.101 | 1.02555 |

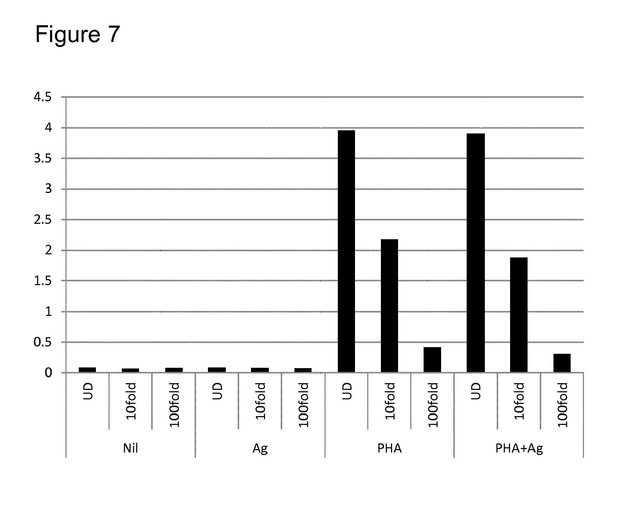

The mean ELISA results from Table 7 are shown graphically in Figure 7. Referring to Table 7 and Figure 7, the results demonstrate that, in the undiluted sample and at each of the dilutions tested, the INF-γ level in the PHA and the PHA + Ag tubes was substantially the same. This indicates that there was no non-specific IFN-γ production by the T cells of HIV-negative/TB non-exposed individuals following co-stimulation with PHA and TB antigens.

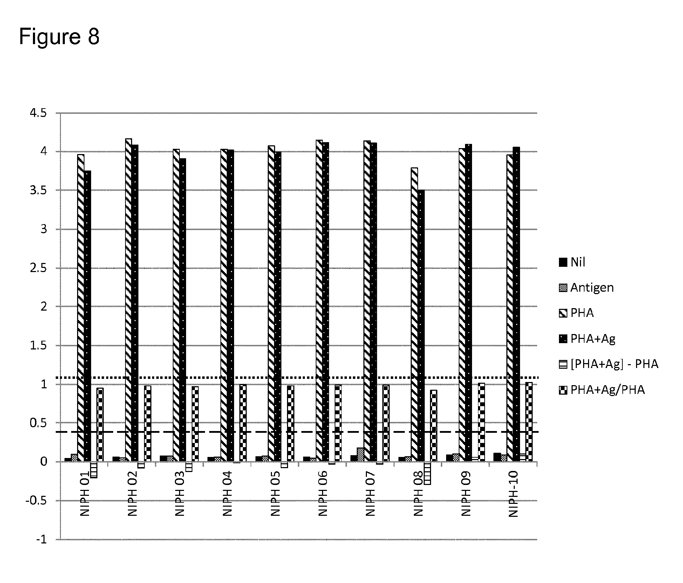

The undiluted values from Table 7 were used to calculate delta and normalised OD values, which are shown in Table 8. The results of Table 8 are shown graphically in Figure 8. Referring to Figure 8, the results show that, using an assumed cut off value of 1.092, none of the 10 HIV-negative/TB non-exposed individuals were detected as TB positive using the normalised OD value. Similarly, using an assumed cut off value of 0.4, none of the 10 HIV-negative/TB non-exposed individuals were detected as TB positive using the delta OD value. These results demonstrate that both the normalised OD and the delta OD approach correctly identify the 10 HIV-negative/TB non-exposed individuals as TB negative.

Conclusion

Co-stimulation of T cells with PHA and TB antigens did not result in non-specific IFN-γ production by T cells from 10 HIV-negative/TB non-exposed individuals. Both the normalised and the delta OD approach correctly identified the10 HIV-negative/TB non-exposed individuals as TB negative.

Claims

**1.**   A method of detecting the presence of lymphocytes reactive with an antigen in an individual, comprising the steps of:

(a) contacting a first lymphocyte sample containing T cells and/or B cells from the individual with: an agent that non-specifically expands and/or activates a T cell and/or a B cell population; and said antigen, in order to generate a first stimulated lymphocyte population; and
(b) detecting in the first stimulated lymphocyte population an indicator of T cell and/or B cell binding to said antigen, wherein the detection of that indicator is indicative of the presence of lymphocytes reactive with the antigen being present in the individual.

**2.**   The method according to claim 1, wherein step (a) further comprises the step of contacting a second lymphocyte sample containing T cells and/or B cells from the individual with the agent in order to generate a second stimulated lymphocyte population and wherein step (b) further comprises the step of detecting in the second stimulated lymphocyte population the indicator of T cell and/or B cell binding to the antigen, and wherein an increase in the level of the indicator in the first stimulated lymphocyte population relative to the second stimulated lymphocyte population is indicative of the presence of lymphocytes reactive with the antigen being present in the individual.

3. The method according to claim 1 or 2, wherein the detection of the presence of lymphocytes reactive with the antigen is indicative of one of the following pathologies in an individual: tuberculosis, leprosy, HIV/AIDS, leishmaniasis, malaria, viral hepatitis, meningitis or cancer.

4. The method according to any one of claims 1 to 3, wherein optical density is used to detect the level of the indicator in the first and/or the second stimulated lymphocyte populations.

5. The method according to claim 4 as dependent on claim 2, wherein a ratio of the optical density of the indicator of the first to the second stimulated lymphocyte population is used to determine the presence of lymphocytes reactive with the antigen in the individual, and wherein a ratio greater than 1 is indicative of the presence of lymphocytes reactive with the antigen being present in the individual.

6. The method according to claim 5, wherein a ratio greater than $1 + k$ is indicative of the presence of lymphocytes reactive with the antigen being present in the individual, and wherein the value of $k$ is 0.092.

7. The method according to any one of the preceding claims, wherein the indicator of T cell binding to the antigen is the production of a cytokine or the production of a mixture of cytokines, preferably wherein the cytokine or one of the cytokines is IFN-$\gamma$.

8. The method according to any one of the preceding claims, wherein the indicator of B cell binding to the antigen is the production of an immunoglobulin or the production of a mixture of immunoglobulins, preferably wherein the immunoglobulin or the immunoglobulins are selected from IgM, IgG, IgA or IgE.

9. The method according to any one of the preceding claims, wherein the agent is a mitogen.

10. The method according to claim 9, wherein the mitogen is phytohaemagglutinin.

11. The method according to any one of the preceding claims, wherein the individual is in an immunocompromised condition or is a child.

12. A kit for detecting the presence of lymphocytes reactive with an antigen in a sample from an individual, comprising:

   (a) an agent that non-specifically expands and/or activates a T cell and/or a B cell population; and
   (b) said antigen.

13. The kit according to claim 12, wherein the agent is a mitogen.

14. The kit according to claim 13, wherein the mitogen is phytohaemagglutinin.

15. The method according to any one of claims 1 to 11, or the kit according to any one of claims 12 to 14, wherein the antigen is any one of, or a cocktail comprising, ESAT-6, CFP-10 or TB 7.7, and wherein the detection of the presence of lymphocytes reactive with said antigens is indicative of tuberculosis in the individual.

Figure 1

Figure 2

Figure 3

Figure 4

## Figure 5

Figure 6

Figure 7

Figure 8

Europäisches
Patentamt

European
Patent Office

Office européen
des brevets

# EUROPEAN SEARCH REPORT

Application Number

EP 15 15 5483

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LOPATIN D E ET AL: "The influence of mitogen costimulation on the human lymphocyte blastogenic response to Actinomyces viscosus ultrasonicates", CLINICAL IMMUNOLOGY AND IMMUNOPATHOLOGY, SAN DIEGO, CA, US, vol. 16, no. 1, 1 May 1980 (1980-05-01), pages 75-83, XP026179550, ISSN: 0090-1229, DOI: 10.1016/0090-1229(80)90168-3 [retrieved on 1980-05-01] | 1 | INV.<br>G01N33/543<br>G01N33/569<br>G01N33/68 |
| Y | * the whole document * | 2-11 | |
| X | WO 2013/175459 A2 (UNIV STELLENBOSCH [ZA]) 28 November 2013 (2013-11-28) | 12-15 | |
| Y | * claims 1-23 *<br>* example 1 * | 2-11 | |
| X | CHRISTINE DANEL ET AL: "Quantiferon-TB Gold: Performance for Ruling out Active Tuberculosis in HIV-Infected Adults with High CD4 Count in Côte d'Ivoire, West Africa", PLOS ONE, vol. 9, no. 10, 16 October 2014 (2014-10-16), page e107245, XP055184661, DOI: 10.1371/journal.pone.0107245<br>* the whole document * | 12-15 | |

TECHNICAL FIELDS
SEARCHED       (IPC)

G01N

The present search report has been drawn up for all claims

| Place of search | Date of completion of the search | Examiner |
|---|---|---|
| Munich | 22 April 2015 | Bayer, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 15 5483

22-04-2015

| Patent document cited in search report | Publication date | Patent family member(s) | Publication date |
|---|---|---|---|
| WO 2013175459 A2 | 28-11-2013 | NONE | |

EPO FORM P0459

**REFERENCES CITED IN THE DESCRIPTION**

**Patent documents cited in the description**

- WO 2010009494 A1

**Non-patent literature cited in the description**

- **GUAR et al.** *PLOS One,* 2012
- **COTTALORDA et al.** *Eur. J. Immunol.,* 2009
- **AABYE et al.** *Clin Vaccine Immunol.,* 2011